# EUROPEAN PATENT SPECIFICATION

(11) **EP 4 450 107 B1**
(45) Date of publication and mention of the grant of the patent: **18.11.2026**
(21) Application number: 22930032.2
(22) Date of filing: 20.11.2022
(51) Int. Cl.: A61M 16/14, A61M 11/04, A61M 16/00, A61M 16/10, A61M 11/00, A61M 16/08, A61M 16/16

(54) **DRUG AEROSOL SUPPLY APPARATUS FOR RESPIRATOR**
ARZNEIMITTELAEROSOLVERSORGUNGSVORRICHTUNG FÜR BEATMUNGSGERÄT
APPAREIL D'ALIMENTATION EN AÉROSOL MÉDICAMENTEUX POUR RESPIRATEUR

(30) Priority: 04.03.2022 KR 20220028215
(43) Date of publication of application: 23.10.2024
(73) Proprietor: Boditech Med Inc., Chuncheon-si, Gangwon-do 24398 (KR)
(72) Inventor: IM, Youn Tae, Chuncheon-si Gangwon-do 24401 (KR); PARK, Sang Hyun, Seoul 07350 (KR); CHOI, Kwang Won, Chuncheon-si Gangwon-do 24209 (KR); SHIN, Jeong Ae, Chuncheon-si Gangwon-do 24399 (KR); KIM, Byeong Chul, Chuncheon-si Gangwon-do 24304 (KR)
(74) Representative: Louis Pöhlau Lohrentz
(86) International application number: PCT/KR2022/018368
(87) International publication number: WO 2023/167388

(56) References cited:
- EP-B1- 2 273 933
- CN-A- 113 424 998
- JP-A- 2013 517 082
- KR-A- 20100 060 004
- KR-B1- 102 361 810
- KR-B1- 102 361 810
- KR-B1- 102 364 175
- KR-B1- 102 364 175
- US-A- 5 762 063
- US-A1- 2014 238 397
- US-A1- 2015 352 299

## Description

### FIELD OF THE INVENTION

The present invention relates to a drug aerosol supply apparatus for a respirator, and more particularly to a drug aerosol supply apparatus that can prevent secondary respiratory infections caused by condensation water.

### BACKGROUND ART

The air generated by a respirator is usually heated to 39°C in a humidifier and humidified to 99% before being provided a drug aerosol supply apparatus. The air heated and humidified in the humidifier is sprayed with a chemical solution in a particulate state and then supplied to the user wearing a mask or a mouthpiece. The drug aerosol supply apparatus includes a T tube, which connects the humidifier and the mask (or the mouthpiece) to a nebulizer.

Because the internal temperature of the T tube of a conventional drug aerosol supply apparatus is relatively low compared to the air heated in the humidifier, moisture condenses and forms droplets on its internal surface. This condensed moisture accumulates inside the T tube over a long period of time and eventually passes into the patient's airway, causing secondary bronchial infections. This situation is further aggravated when the chemical solution is sprayed in a particulate state by the drug aerosol supply apparatus. The document KR 102 364 175 B1 represents relevant prior art with respect to the present invention.

### DETAILD DESCRIPTION OF THE INVENTION

### TECHNICAL PROBLEM

Accordingly, one objective of the present invention is to prevent moisture from condensing inside the drug aerosol supply apparatus.

Furthermore, another objective of the present invention is to prevent moisture condensed in the drug aerosol supply apparatus from passing into the patient's airway and causing secondary bronchial infections.

### TECHNICAL SOLUTION

One aspect of the present invention to achieve the foregoing objectives is a drug aerosol supply apparatus for a respirator according to claim 1, the device comprising: a T tube including a straight tube having a first end portion to which air provided from a respirator is provided and having a second end portion connected to a mask or a mouthpiece, and a branch tube attached to the straight tube so as to allow communication with the straight tube; a drug particulate generation unit, which is installed at the branch tube and sprays a drug in a particulate state; and a heater for heating the T tube.

The heater comprises a film including a heating wire and is attached to the T tube. In addition, the heater includes a temperature sensor that measures the temperature of the T tube. In addition, the heater is integrally formed with a first local heater that heats the straight tube and a second local heater that heats the branch unit. A heating wire terminal for connecting the heating wire to an external power source is disposed at the second end portion.

The drug aerosol supply apparatus further includes a socket unit including a heating wire connection terminal electrically connected to the heating wire terminal and a power connection terminal electrically connected to the external power source. The power connection terminal is arranged to face the first end portion of the straight tube. The socket unit is installed on the straight tube so that the power connection terminal is inclined downward with respect to the straight tube. Additionally, the socket unit has a sensor escape groove in which the temperature sensor escapes. The heating wire connection terminal is connected to the power connection terminal via an elastic body.

### TECHNICAL EFFECTS

The present invention can prevent moisture from condensing inside the drug aerosol supply apparatus. In addition, the present invention can prevent moisture condensed in the drug aerosol supply apparatus from passing into the patient's airway and causing secondary bronchial infections.

### BRIEF DESCRIPTION OF THE DRAWINGS

FIG. 1 is a view illustrating the configuration of a respirator to which a drug aerosol supply apparatus according to an embodiment of the present invention is applied.
FIG. 2 is a cross-sectional view illustrating the function of the drug aerosol supply apparatus shown in FIG. 1.
FIG. 3 is a view illustrating the configuration of a drug aerosol supply apparatus to which an embodiment of the present invention is applied.
FIG. 4 is a view illustrating the configuration of the T tube, the heater, and the socket unit shown in FIG. 3.
FIG. 5 is a view illustrating the assembly process of the T tube, the heater, and the socket unit shown in FIG. 3.
FIG. 6A is a view illustrating the configuration for assembling the socket unit to the T tube to which the heater shown in FIG. 3 is attached, and FIG. 6B is a view illustrating the assembled state.
FIG. 7 is a view illustrating a state in which a power cable is connected to the drug aerosol supply apparatus shown in FIG. 3.

### BEST MODE FOR ACCOMPLISHING THE INVENTION

Hereinafter, embodiments of the present invention will be described in detail with reference to the accompanying drawings. Terms used in this description should not be construed as being limited to conventional or dictionary meanings. Therefore, the embodiments described in this specification and the configurations shown in the drawings are only one of the most preferred embodiments of the present invention and do not represent all of the technical ideas of the present invention.

In the entire drawing, those having the same function are given the same reference numerals, and detailed description thereof will be omitted.

Meanwhile, terms such as "first" and "second" may be used to describe various components, and the above terms are used only for the purpose of distinguishing one component from other components.

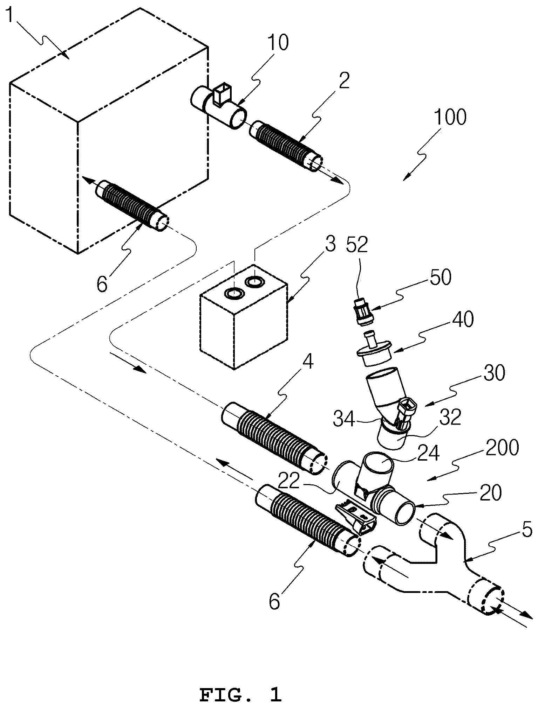

FIG. 1 is a view illustrating a configuration of a respirator 100 to which a drug aerosol supply apparatus 200 according to an embodiment of the present invention is applied. As shown in FIG. 1, the respirator 100 includes an air purifier 1, an intake breathing detector 10, a T tube 20, a cover 40, and a packing closure 50.

The air purifier 1 discharges first air having a concentration of oxygen and a pressure of oxygen and a velocity of oxygen each set to a predetermined value. The oxygen concentration may be set according to the patient's lung function ability. For example, if the lung function is half the level of the normal person, the oxygen concentration may be set to approximately 40%. The intake breathing detector 10 may be connected to the air purifier 1. The intake breathing detector 10 may detect the first air and generate a detection signal when the first air is discharged from the intake breathing detector 10. The first tube 2 is connected to the intake breathing detector 10 and allows the first air to flow. A humidifier 3 is connected to the first tube 2 and controls the temperature and humidity of the first air to generate second air and then discharge it. The second tube 4 is connected to the humidifier 3 and allows the second air to flow. The T tube 20 has a branch tube 24 formed on one side of the straight tube 22. The second tube 4 is connected to one end of the straight tube 22, so that the second air can pass through the straight tube 22. The drug particulate generation unit 30 is installed at the branch tube 24 and operates to spray the drug in a particulate state into the second air only when the detection signal is input.

The Y tube 5 may have one end portion connected to the T tube 20 and another end portion connected to a mask or a mouthpiece. The return tube 6 has one end portion connected to the Y tube 5 and the other end portion connected to the air purifier 1 to allow exhaled breathing air to flow.

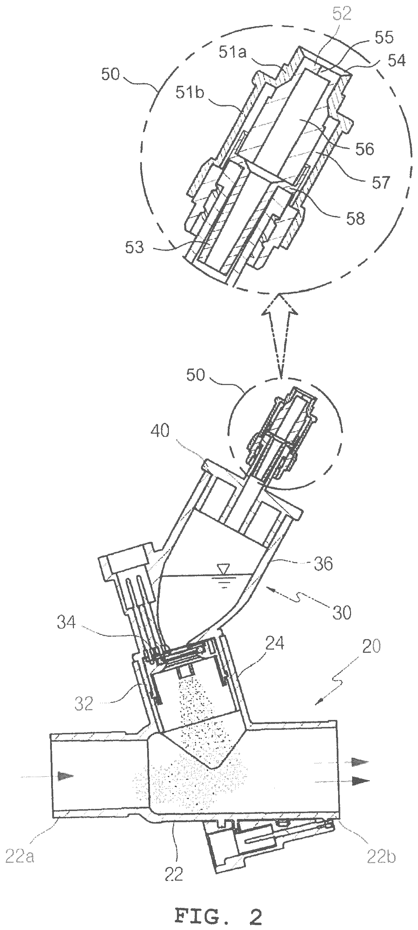

FIG. 2 is a cross-sectional view illustrating the function of the drug aerosol supply apparatus 200 shown in FIG. 1.

In the drug aerosol supply apparatus 200, a drug particulate generation unit 30 is disposed at the inlet of the Y tube 5. The drug particulate generation unit 30 changes the drug into a particulate state by ultrasonic vibration. Thereby, the distance from the location where the drug in the aerosol state is generated to the mask (or the mouthpiece) can be as short as possible. In other words, the path for the drug in particulate state to travel is very short. Therefore, a good drug effect can be expected even if a small amount of the drug is changed to a particulate state.

The drug aerosol supply apparatus 200 does not additionally introduce external air. This makes it possible to maintain the oxygen concentration, oxygen pressure, and oxygen velocity of the first air treated by the air purifier 1 and the second air treated by the humidifier 3, and thus the patient can breathe more naturally. In addition, the drug aerosol supply apparatus 200 operates the drug particulate generation unit 30 according to the detection signal generated from the intake breathing detector 10, so that the drug can be accurately sprayed at the time the drug is consumed. As a result, the drug aerosol supply apparatus 200 can significantly reduce drug waste.

In addition to detecting the first air discharged from the air purifier 1, the detection signal for operating the drug particulate generation unit 30 in the drug aerosol supply apparatus 200 may be diversified as follows. 1) The detection signal may be generated by a chest sensor installed on a patient's chest that detects when the patient's chest expands. When the patient breathes, the chest rises and falls repeatedly. Such movements can be detected and used as a detection signal. 2) The detection signal may be a specific brain wave signal generated when a patient breathes. When the lung organs operate, specific brain waves are generated in the brain, and those brain waves can be detected and used as a detection signal. 3) The detection signal may be generated by installing a sensor on a mask or a mouthpiece to detect air flow during inhalation. There is a difference in the direction of the air flow between the inhalation and the exhalation, and such a difference may be detected and used as a detection signal. 4) The detection signal may be generated by installing a sensor on a mask or a mouthpiece to detect air pressure during inhalation. There is a difference between the pressure in inhalation and the pressure in exhalation. Such a difference may be detected and used as a detection signal. 5) The detection signal may be generated by installing a sensor on a mask or a mouthpiece to detect the air temperature during inhalation. There is a difference between the temperature in inhalation and the temperature in exhalation. Such a difference may be detected and used as a detection signal. That is, the drug aerosol supply apparatus 200 according to other embodiments of the present invention may replace the detection signal generated by the intake breathing detector 10 with at least one of the various examples described above. Additionally, by applying all of the various examples described above, the drug particulate generation unit 30 can be operated when any one detection signal is generated.

As illustrated in FIG. 2, the drug particulate generation unit 30 may include an intubation 32, a particulate generating element 34, a medicine container 36, a cover 40, and a packing closure 50. The intubation 32 is assembled to the branch tube 24. The particulate generating element 34 may be placed on top of the intubation 32. The particulate generating element 34 can change the drug into a particulate state using, for example, ultrasonic vibration. Since this technology is well known, further detailed description will be omitted. The medicine container 36 is disposed above the particulate generating element 34 to store the drug. The cover 40 is placed on the medicine container 36, thereby preventing the drug stored in the medicine container 36 from being exposed to the outside.

The packing closure 50 may be installed at the cover 40. The packing closure 50 includes an upper case 51a, a lower case 51b, a packing 52 and a discharge portion 53. The upper case 51a has an inner diameter smaller than that of the lower case 51b. The upper case 51a has an aperture 54 formed at the top, and a syringe or the like can be inserted into the aperture 54. The lower portion of the upper case 51a is connected with the upper portion of the lower case 51b. The lower portion of the lower case 51b is connected to the discharge portion 53. The packing 52 is made of an elastic material, such as soft rubber, and is placed inside both the upper case 51a and the lower case 51b. The packing 52 has an incision 55 on the top or side, and a passage 56 formed therein. The passage 56 connects the incision 55 and the discharge portion 53. The packing 52 has an external shape that seals the inner wall of the upper case 51a and has a slight gap 57 with the inner wall of the lower case 51b. The lower case 51b has a threshold portion 58 at the bottom, and the threshold portion 58 supports the bottom of the packing 52.

The packing 52 normally seals the packing closure 50 (especially the inner wall of the upper case 51a). However, for example, if a syringe (not shown) without a needle is inserted through the aperture 54, the threshold portion 58 supports the packing 52 from backing out and the gap 57 allows the packing 52 to be deformed, so the top of the packing 52 is pushed back and the incision 55 is opened. In this state, when the syringe injects the drug, the drug is filled in the medicine container 36 via the incision 55, the passage 56, and the discharge portion 53. When the syringe is separated from the packing closure 50, the packing closure 50 is immediately sealed due to the elasticity of the packing 52. Accordingly, since the inside of the medicine container 36 can be fundamentally blocked from being exposed to the outside, contamination of the medicine container 36 can be prevented. Therefore, patients can be treated in a more strictly clean environment. In addition, by preventing the inside of the medicine container 36 from being exposed, it is possible to strictly prevent the inside of the entire section of the respirator, especially the entire section of the tube connected from the air purifier 1 to the mask (or mouthpiece) from being exposed to the outside.

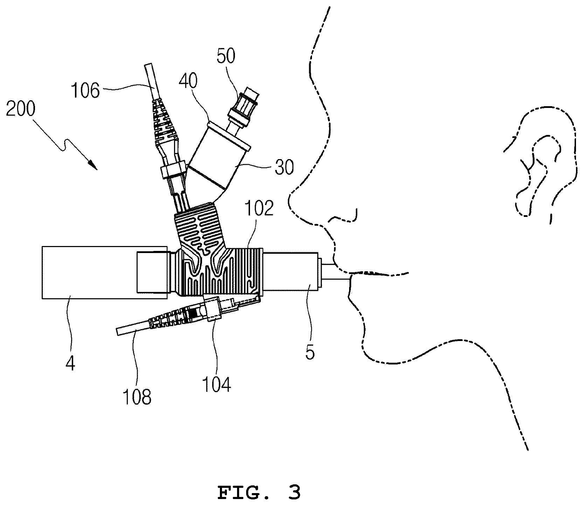

FIG. 3 is a diagram illustrating the configuration of the drug aerosol supply apparatus 200 to which an embodiment of the present invention is applied. As shown, the drug aerosol supply apparatus 200 includes a T tube 20, a drug particulate generation unit 30, and a heater 102.

The T tube 20 includes a straight tube 22 and a branch tube 24. Air provided from the humidifier 3 is provided through the tube 4 at the first end portion 22a of the straight tube 22, and the second end portion 22b is connected to a mask or a mouthpiece via a Y tube 5. The branch tube 24 is attached to the straight tube 22 and communicates with the straight tube 22. The drug particulate generation unit 30 is installed at the branch tube 24 and sprays the drug in a particulate state. The heater 102 heats the T tube 20.

The heater 102 is attached to the outer surface of the T tube 20 by a method such as adhesion, and the heater 102 heats the T tube 20 so that the temperature of the inner surface of the T tube 20 is similar to the temperature of the second air discharged from the humidifier 3. When the second air passes through the T tube 20 heated by the heater 102, the moisture vaporized in the second air is not condensed. Therefore, secondary respiratory infections due to condensation water can be prevented.

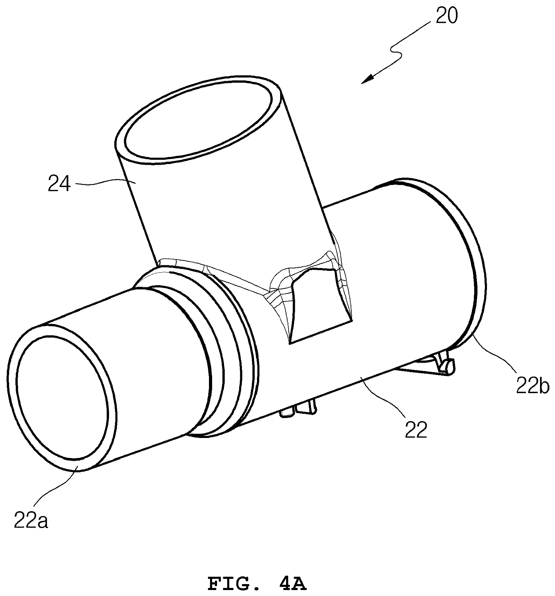

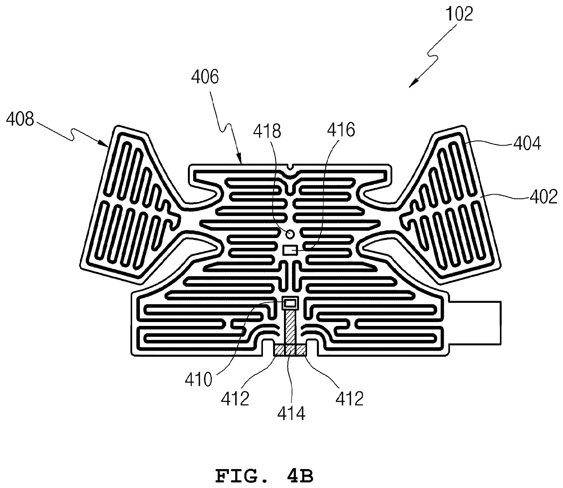

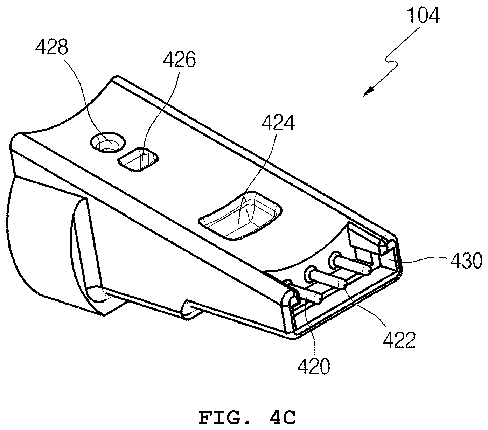

FIG. 4A illustrates the configuration of the T tube 20 before being assembled, FIG. 4B illustrates the configuration of the heater 102, and FIG. 4C illustrates the configuration of the socket unit.

It is preferable to see the inside of the T tube 20 to check whether spray and condensate are generated from the drug particulate generation unit 30. For this purpose, the T tube 20 and the film 402 are made of a transparent or translucent material.

The heater 102 comprises the film 402 including a heating wire 404 and is attached to the outer surface of the T tube 20. The heater 102 includes a first local heater 406 for heating the straight tube 22 and a second local heater 408 for heating the branch tube 24. The heater 102 includes a temperature sensor 410 for measuring the temperature of the T tube 20. The temperature sensor 410 is for monitoring and controlling the temperature of the T tube 20, and is disposed approximately at the center of the heater 102.

The heater 102 has a heating wire terminal 412 for connecting the heating wire 404 to an external power source, and a sensor terminal 414 for connecting the temperature sensor 410 to an external control device. The heating wire terminal 412 and the sensor terminal 414 are attached to the second end portion 22b. In addition, the heater 102 includes a hook hole 416 and a boss hole 418 for connecting the T tube 20 and the socket unit 104.

The socket unit 104 includes a heating wire connection terminal 420 electrically connected to the heating wire terminal 412 and a sensor connection terminal 422 electrically connected to the sensor terminal 414. The socket unit 104 has a sensor escape groove 424, a hook connection groove 426, and a boss connection groove 428 formed on an upper surface attached to the T tube 20. Additionally, the socket unit 104 has a locking jaw 430 at the front portion.

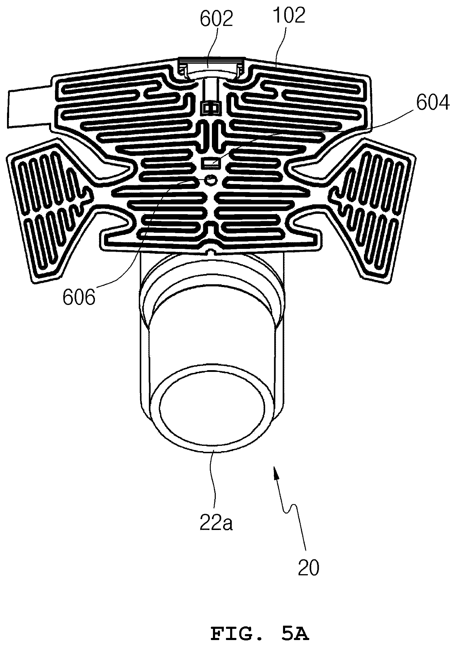

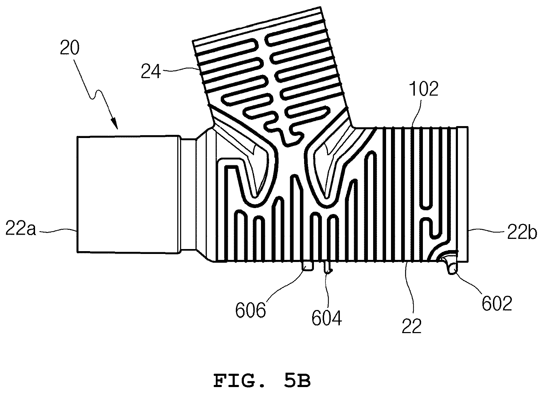

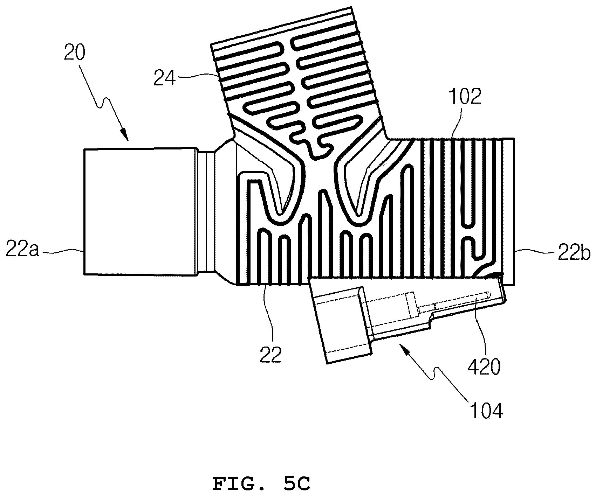

FIG. 5A to FIG. 5C illustrate assembling the T tube 20, the heater 102, and the socket unit 104 shown in FIG. 3. First, as shown in FIG. 5A, the heater 102 is attached from the lower surface of the straight tube 22 of the T tube 20, and as shown in FIG. 5B, the heater 102 is attached to the branch tube 24. Next, as shown in FIG. 5C, the socket unit 104 is assembled to the straight tube 22 of the T tube 20.

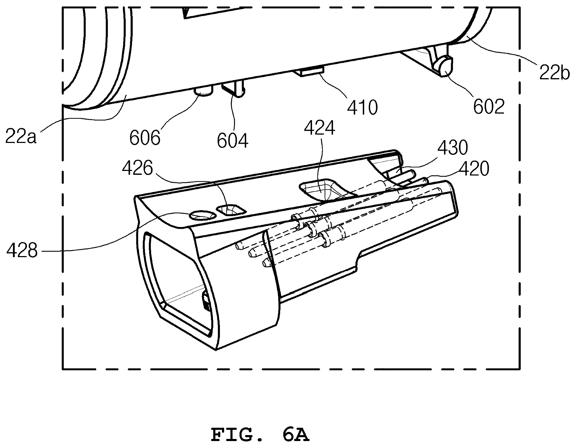

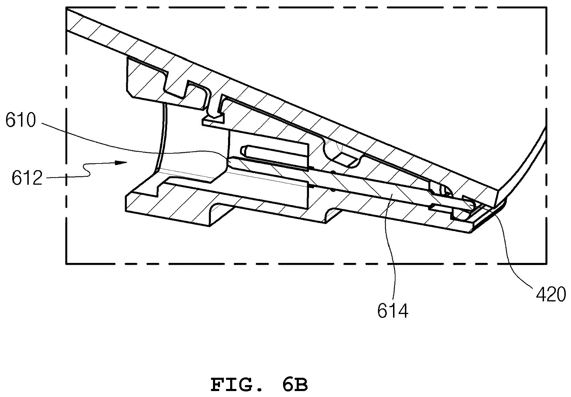

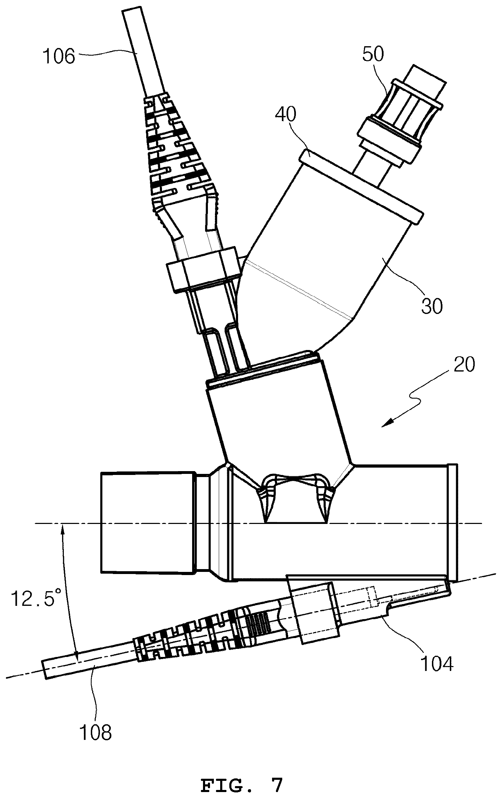

FIG. 6A illustrates the configuration for assembling the socket unit 104 to the T tube 20 to which the heater 102 shown in FIG. 3 is attached, and FIG. 6B illustrates the state in which the assembling is completed. FIG. 7 is a view for illustrating the state in which a power cable is connected to the drug aerosol supply apparatus 200 shown in FIG. 3.

Since the power cable 108 and the heater 102 cannot be directly connected, they are electrically connected via the socket unit 104. The power cable 108 may include a reverse assembly prevention structure to prevent reverse assembly when connected to the cable socket 612.

The heater 102 is attached to the outer surface of the T tube 20, and the temperature sensor 410 provided in the heater 102 is located in the sensor escape groove 424. Accordingly, the temperature sensor 410 is sealed by the T tube 20 and the socket unit 104, and thus is protected from external impact.

The socket unit 104 includes a power connection terminal 610 electrically connected to an external power source. The power connection terminal 610 is exposed to the cable socket 612. When the patient wears a mask or a mouthpiece, the power cable 108 should not be directed to the patient. Therefore, the cable socket 612 is disposed on the opposite side of the Y tube 5 with respect to the T tube 20. Accordingly, the power connection terminal 610 is arranged to face the first end portion 22a of the straight tube 22 while the socket unit 104 is assembled to the T tube 20.

The socket unit 104 is configured such that the power connection terminal 610 is inclined downward, for example, approximately 12.5 degrees with respect to the straight tube 22, to facilitate fastening of the power cable 108.

The power connection terminal 610 and the heating wire connection terminal 420 may be the end portions of the pogo pin 614. The pogo pin 614 has a spring inside to support the heating wire connection terminal 420. Therefore, in the process of fastening the socket unit 104 to the T tube 20, the fastening can be performed smoothly, and after fastening, the electrical connection between the heating wire connection terminal 420 and the heating wire terminal 412 can be firmly maintained.

The T tube 20 has a socket locking protrusion 602, a hook 604, and a boss fitting portion 606 on the lower surface to which the socket unit 104 is fastened. The socket locking protrusion 602 is formed at the second end portion 22b of the straight tube 22 and is fastened to the locking jaw 430 of the socket unit 104. The socket locking protrusion 602 and the locking jaw 430 allow the heating wire connection terminal 420 of the pogo pin 614 to be connected to the heating wire terminal 412, and the sensor connection terminal 422 to be connected to the sensor terminal 414. The hook 604 is fastened to the hook connection groove 426 via the hook hole 416. The hook 604 is fastened to the hook connection groove 426 to prevent the socket unit 104 from being separated from the T tube 20 and prevent the socket unit 104 from moving by being pushed in the opposite direction by the elasticity of the pogo pin 614. In addition, the boss fitting portion 606 is fastened to the boss connection groove 428 via the boss hole 418. The boss fitting portion 606, along with the hook 604, prevents the socket unit 104 from moving by being pushed in the opposite direction by the elasticity of the pogo pin 614.

In the present embodiment, the heater 102 has a film including a heating wire 404 and is assembled to the T tube 20 in an attachment manner. In addition, since the screw is not used when assembling the socket unit 104 to the T tube 20, the assembly procedure can be minimized.

The scope of the invention is limited by the scope of the appended claims.

## Claims

1. A drug aerosol supply apparatus for a respirator, the apparatus comprising:
a T tube (20) including a straight tube (22) having a first end portion (22a) to which air provided from a respirator is provided and having a second end portion (22b) connected to a mask or a mouthpiece, and a branch tube (24) attached to the straight tube (22) so as to allow communication with the straight tube (22);
a drug particulate generation unit (30), which is installed at the branch tube (24) and sprays a drug in a particulate state; and
a heater (102) for heating the T tube (20);
**characterized in that**:
the heater (102) comprises a film (402) including a heating wire (404) and is attached to the outer surface of the T tube (20);
wherein the heater (102) includes a first local heater (406) for heating the straight tube (22) and a second local heater (408) for heating the branch tube (24), and the first local heater and the second local heater are integrally formed;
wherein the heater (102) further includes a heating wire terminal (412) disposed at the second end portion (22b) and connecting the heating wire (404) to an external power source;
the apparatus further comprising a socket unit (104) including a heating wire connection terminal (420) electrically connected to the heating wire terminal (412) and a power connection terminal (610) electrically connected to the external power source;
wherein the power connection terminal (610) and the heating wire connection terminal (420) are end portions of a pogo pin (614), the pogo pin having a spring inside to support the heating wire connection terminal (420); and
wherein the socket unit (104) is assembled to the T tube (20) without the use of screws by fastening the locking jaw (430) of the socket unit (104) to the T tube' s socket locking protrusion (602), and by engaging the socket unit's hook connection groove (426) with the T tube's hook (604), and by engaging the socket unit's boss connection groove (428) with the T tube's boss fitting portion (606).

2. The drug aerosol supply apparatus of claim 1, wherein the heater (102) further comprises a temperature sensor (410) that measures the temperature of the T tube (20).

3. The drug aerosol supply apparatus of claim 1, wherein the socket unit (104) has a sensor escape groove (424) in which the temperature sensor (410) is located and sealed by the T tube (20) and the socket unit (104) to be protected from external impact.

4. The drug aerosol supply apparatus of claim 1, wherein the T tube (20) and the film (402) are made of a transparent or translucent material so that the spray and condensate generated from the drug particulate generation unit (30) can be visually observed.

## Patentansprüche

1. Arzneimittel-Aerosol-Abgabevorrichtung für ein Beatmungsgerät, die Vorrichtung umfasst:
ein T-Rohr (20) umfassend ein gerades Rohr (22), das einen ersten Endabschnitt (22a), an den aus einem Beatmungsgerät zugeführte Luft zugeführt wird, und einen zweiten Endabschnitt (22b) aufweist, der mit einer Maske oder einem Mundstück verbunden ist, und ein Abzweigrohr (24), das so am geraden Rohr (22) angebracht ist, dass eine Verbindung mit dem geraden Rohr (22) ermöglicht ist;
eine Arzneimittelpartikelerzeugungseinheit (30), die am Abzweigrohr (24) angebracht ist und ein Arzneimittel in einem partikelförmigen Zustand versprüht; und
eine Heizvorrichtung (102) zum Erwärmen des T-Rohrs (20);
**dadurch gekennzeichnet, dass**:
die Heizvorrichtung (102) eine Folie (402) mit einem Heizdraht (404) umfasst und an der Außenfläche des T-Rohrs (20) angebracht ist;
wobei die Heizvorrichtung (102) eine erste lokale Heizvorrichtung (406) zum Erwärmen des geraden Rohrs (22) und eine zweite lokale Heizvorrichtung (408) zum Erwärmen des Abzweigrohrs (24) umfasst und die erste lokale Heizvorrichtung und die zweite lokale Heizvorrichtung integral geformt sind;
wobei die Heizvorrichtung (102) ferner einen Heizdrahtanschluss (412) umfasst, der am zweiten Endabschnitt (22b) angeordnet ist und den Heizdraht (404) mit einer externen Stromquelle verbindet;
wobei die Vorrichtung ferner eine Buchseneinheit (104) umfasst, die einen Heizdrahtverbindungsanschluss (420), der elektrisch mit dem Heizdrahtanschluss (412) verbunden ist, und einen Stromverbindungsanschluss (610) umfasst, der elektrisch mit der externen Stromquelle verbunden ist;
wobei der Stromverbindungsanschluss (610) und der Heizdrahtverbindungsanschluss (420) Endabschnitte eines Pogo-Pins (614) sind, der Pogo-Pin im Inneren eine Feder aufweist, um den Heizdrahtverbindungsanschluss (420) zu stützen; und
wobei die Buchseneinheit (104) an dem T-Rohr (20) ohne die Verwendung von Schrauben montiert wird, indem die Verriegelungsklemme (430) der Buchseneinheit (104) an dem Buchsenverriegelungsvorsprung (602) des T-Rohrs befestigt wird und indem die Hakenverbindungskerbe (426) der Buchseneinheit mit dem Haken (604) des T-Rohrs ineinandergreift, und indem die Nabenverbindungskerbe (428) der Buchseneinheit mit dem Nabeneinbauabschnitt (606) des T-Rohrs ineinandergreift.

2. Arzneimittel-Aerosol-Abgabevorrichtung nach Anspruch 1, wobei die Heizvorrichtung (102) ferner einen Temperatursensor (410) umfasst, der die Temperatur des T-Rohrs (20) misst.

3. Arzneimittel-Aerosol-Abgabevorrichtung nach Anspruch 1, wobei die Buchseneinheit (104) eine Sensorauslasskerbe (424) aufweist, in der sich der Temperatursensor (410) befindet und durch das T-Rohr (20) und die Buchseneinheit (104) abgedichtet ist, um vor äußerem Einflussgeschützt zu sein.

4. Arzneimittelaerosol-Abgabevorrichtung nach Anspruch 1, wobei das T-Rohr (20) und die Folie (402) aus einem transparenten oder durchscheinenden Material bestehen, sodass der Sprühnebel und das Kondensat, die von der Arzneimittelpartikelerzeugungseinheit (30) erzeugt werden, visuell beobachtet werden können.

## Revendications

1. Un appareil d'alimentation en aérosol médicamenteux pour un respirateur, l'appareil comprenant :
un tube en T (20) comprenant un tube droit (22) ayant une première partie d'extrémité (22a) à laquelle l'air fourni par un respirateur est fourni et ayant une deuxième partie d'extrémité (22b) connectée à un masque ou à un embout buccal, et un tube de dérivation (24) fixé au tube droit (22) de manière à permettre la communication avec le tube droit (22) ;
une unité de génération de particules de médicament (30), qui est installée au niveau du tube de dérivation (24) et pulvérise un médicament à l'état particulaire ; et
un dispositif de chauffage (102) pour chauffer le tube en T (20) ;
**caractérisé en ce que** :
le dispositif de chauffage (102) comprend un film (402) comprenant un fil chauffant (404) et est fixé à la surface externe du tube en T (20) ;
dans lequel le dispositif de chauffage (102) comprend un premier dispositif de chauffage local (406) pour chauffer le tube droit (22) et un deuxième dispositif de chauffage local (408) pour chauffer le tube de dérivation (24), et le premier dispositif de chauffage local et le deuxième dispositif de chauffage local sont formés de manière solidaire ; dans lequel le dispositif de chauffage (102) comprend en outre une borne de fil chauffant (412) disposée à la deuxième partie d'extrémité (22b) et connectant le fil chauffant (404) à une source d'alimentation externe ;
l'appareil comprenant en outre une unité de prise (104) comprenant une borne de connexion de fil chauffant (420) électriquement connectée à la borne de fil chauffant (412) et une borne de connexion d'alimentation (610) électriquement connectée à la source d'alimentation externe ;
dans lequel la borne de connexion d'alimentation (610) et la borne de connexion de fil chauffant (420) sont des parties d'extrémité d'une broche de contact à ressort (614), la broche de contact à ressort ayant un ressort à l'intérieur pour supporter la borne de connexion de fil chauffant (420) ; et
dans lequel l'unité de prise (104) est assemblée au tube en T (20) sans utilisation de vis en fixant la mâchoire de verrouillage (430) de l'unité de prise (104) à la saillie de verrouillage de prise (602) du tube en T, et en mettant en prise la rainure de connexion à crochet (426) du crochet (604) du tube en T, et en mettant en prise la rainure de connexion de bossage (428) de l'unité de prise avec la partie d'ajustement de bossage (606) du tube en T.

2. L'appareil d'alimentation en aérosol médicamenteux selon la revendication 1, dans lequel le dispositif de chauffage (102) comprend en outre un capteur de température (410) qui mesure la température du tube en T (20).

3. L'appareil d'alimentation en aérosol médicamenteux selon la revendication 1, dans lequel l'unité de prise (104) présente une rainure d'échappement de capteur (424) dans laquelle le capteur de température (410) est situé et scellé par le tube en T (20) et l'unité de prise (104) afin d'être protégé contre les chocs extérieurs.

4. L'appareil d'alimentation en aérosol médicamenteux selon la revendication 1, dans lequel le tube en T (20) et le film (402) sont constitués d'un matériau transparent ou translucide de sorte que la pulvérisation et le condensat générés par l'unité de génération de particules de médicament (30) puissent être observés visuellement.
